# EUROPEAN PATENT APPLICATION

(11) **EP 1 354 586 A1**
(43) Date of publication of application: **22.10.2003**
(21) Application number: 02008902.5
(22) Date of filing: 20.04.2002
(51) Int. Cl.: A61K 31/4412, A61K 31/4418, A61K 31/4427, A61K 31/721, A61K 31/715, A61K 31/44

(54) **The use of hydroxpyridone-derivatives in wound healing**

(71) Applicant: Aventis Pharma Deutschland GmbH, 65929 Frankfurt am Main (DE)
(72) Inventor: Bohn, Manfred Dr., 65719 Hofheim (DE); Kraemer, Karl Theodor Dr., 63225 Langen (DE); Wenger, Roland H. Dr., CH-8050 Zürich-Oerlikon (CH)

(57) **Abstract**

The present invention relates to the use of compounds of the formula I, 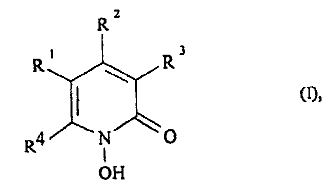 in which R¹; R²; R³ and R⁴ have the meanings indicated in the claims, for the treatment of wounds.

## Description

The present invention relates to the use of compounds of the formula I, 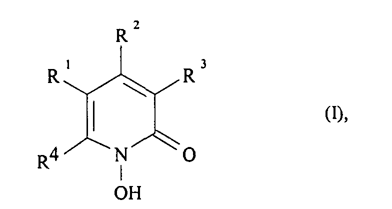 in which R¹; R²; R³ and R⁴ have the meanings indicated below, in wound healing.

Compounds of formula I such as 6-Cyclohexyl-1-hydroxy-4-methyl-2(1H)-pyridone 2-aminoethanol salt, also known as ciclopirox olamine (CPX) are widespread anti-fungal agents used to treat mycoses of the skin and nails since more than 20 years. Apart from its antimycotic activity, CPX is also effective against both gram-positive and gram-negative bacteria (Dittmar, W. et al.(1981) Microbiological laboratory studies with ciclopiroxolamine. *Arzneimittelforschung* **31,** 1317-1322). CPX is applied as a 1 % cream or gel to treat dermatomycoses and as a 8% nail lacquer to treat onychomycoses. CPX is known to inhibit diverse fungal enzymatic activities in microorganisms, including the transmembrane transport of amino acids, potassium and phosphate. However, the general molecular mechanism of the anti-microbial activity of CPX is not exactly known. In studies addressing the cutaneous effects of a 1% CPX solution applied on rabbit skin over 20 days, occasionally transient reddening of healthy skin and persistent reddening of experimentally wounded skin has been observed (Alpermann, H. G., and Schütz, E. (1981) Studies on the pharmacology and toxicology of ciclopiroxolamine. *Arzneimittelforschung* **31,** 1328-1332). Occasionally transient reddening of healthy skin was explained as a side reaction of CPX.

Therapeutic angiogenesis by recombinant angiogenic growth factors or by gene therapy has become an important treatment modality during the past few years (Höckel, M. et al. (1993) Therapeutic angiogenesis. *Arch. Surg*. **128,** 423-429). In ongoing human clinical trials, VEGF gene therapy is applied for the treatment of critical limb ischemia and myocardial ischemia. VEGF therapy has also been experimentally investigated in gastric and duodenal ulceration, as well as in dermal ulcers and in cultured skin substitutes.

It has now been found that the compounds of the formula I have a potent angiogenic activity and induce angiogenesis as evidenced by numerous newly formed arranged vessels. Additionally, it has been found that topic application of CPX does not lead to gross systemic alterations in VEGF expression, which limits its function to regions of topical application. This is rather safe and highly advantageous compared to gene therapy.

The invention therefore relates to the use of 1-hydroxy-2-pyridones of the formula I 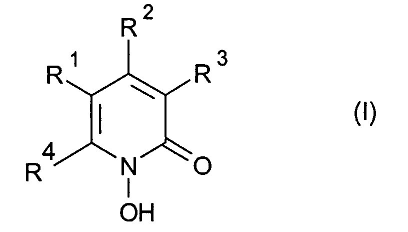 for the preparation of a pharmaceutical for the induction of angiogenesis,
in which R¹, R² and R³ are identical or different and are a hydrogen atom or alkyl having 1 to 4 carbon atoms, and
- R⁴ is: a saturated hydrocarbon radical having 6 to 9 carbon atoms or a radical of the formula II 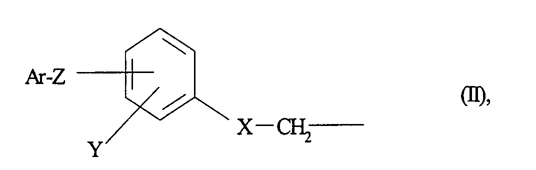 where
- X: is S or O,
- Y: is a hydrogen atom or up to 2 halogen atoms such as chlorine and/or bromine,
- Z: is a single bond or the divalent radicals O, S, -C(R⁵)(R⁶)-; wherein R⁵ and R⁶ are identical or different and are hydrogen atom or (C₁-C₄)-alkyl; or other divalent radicals having 2-10 carbon and optionally O and/or S atoms linked in the form of a chain, where - if the radicals contain 2 or more O and/or S atoms - the latter must be separated from one another by at least 2 carbon atoms and where 2 adjacent carbon atoms can also be linked to one another by a double bond and the free valences of the carbon atoms are saturated by hydrogen atom and/or (C₁-C₄)-alkyl groups,
- Ar: is an aromatic ring system having up to two rings which can be substituted by up to three radicals from the group consisting of fluorine, chlorine, bromine, methoxy, (C₁-C₄)-alkyl, trifluoromethyl and trifluoromethoxy in free or in salt form.

In the radicals "Z", the carbon chain members are preferably CH₂ groups. If the CH2 groups are substituted by C₁-C₄ alkyl groups, CH₃ and C₂H₅ are preferred substituents.

Exemplary radicals of "Z" are:
-O-, -S-, -CH₂-, -(CH₂)ₘ- (m = 2 - 10), -C(CH₃)₂-, -CH₂O-, -OCH₂-, -CH₂S-, -SCH₂-, -SCH(C₂H₅)-, -CH=CH-CH₂O-, -O-CH₂-CH=CH-CH₂O-, -OCH₂-CH₂O-, -OCH₂-CH₂CH₂O-, -SCH₂CH₂CH₂S-, -SCH₂CH₂CH₂CH₂O-, -SCH₂CH₂OCH₂CH₂O-, -SCH₂CH₂OCH₂CH₂O-CH₂CH₂S- or -S-CH₂-C(CH₃)₂-CH₂-S-.

The radical "S" denotes a sulphur atom; the radical "O" denotes an oxygen atom. The term "Ar" denotes phenyl or condensed systems such as naphthyl, tetrahydronaphthyl and indenyl, and also isolated systems such as those, which are derived from biphenyl, diphenylalkanes, diphenyl ethers and diphenyl thioethers.

The term "saturated hydrocarbon radical having 6 to 9 carbon atoms" means that the radical can be straight-chain, branched or cyclic and contains no aliphatic multiple bonds, i.e. no ethylenic or acetylenic bonds. Examples of the R⁴ radical are e.g. hexyl, heptyl, octyl, cyclohexyl or cycloheptyl.

The hydrocarbon radical R⁴ in the compound of formula I is preferably an -(C₆-C₈)-alkyl or cyclohexyl radical, which may also be linked via a methylene or ethylene group to the pyridone ring or can contain an endomethyl group. R⁴ can also be an aromatic radical which, however, is preferably bonded to the pyridone radical via at least one aliphatic carbon atom.

Important representatives of the class of compound characterised by the formula I are:
6-[4-(4-chlorophenoxy)phenoxymethyl]-1-hydroxy-4-methyl-2-pyridone,
6-[4-(2,4-dichlorophenoxy)phenoxymethyl]-1-hydroxy-4-methyl-2-pyridone,
6-(biphenylyl-4-oxymethyl)-1-hydroxy-4-methyl-2-pyridone,
6-(4-benzyl-phenoxymethyl)-1-hydroxy-4-methyl-2-pyridone,
6-[4-(2,4-dichloro-benzyloxy)phenoxymethyl]-1-hydroxy-4-methyl-2-pyridone,
6-[4-(4-chlorophenoxy)phenoxymethyl]-1-hydroxy-3,4-dimethyl-2-pyridone,
6-[4-(2,4-dichlorobenzyl)phenoxymethyl]-1-hydroxy-3,4-dimethyl-2-pyridone,
6-[4-(cinnamyloxy)phenoxymethyl]-1-hydroxy-4-methyl-2-pyridone,
1-hydroxy-4-methyl-6-[4-(4-trifluoromethylphenoxy)phenoxymethyl]-2-pyridone,
1-hydroxy-4-methyl-6-cyclohexyl-2-pyridone,
1-hydroxy-4-methyl-6-(2,4,4-trimethylpentyl)-2-pyridone,
1-hydroxy-4-methyl-6-n-hexyl-, -6-iso-hexyl-, -6-n-heptyl- or -6-iso-heptyl-2-pyridone,
1-hydroxy-4-methyl-6-octyl- or -6-iso-octyl-2-pyridone, in particular 1-hydroxy-4-methyl-6-cyclohexyl-methyl- or -6-cyclohexylethyl-2-pyridone, where the cyclohexyl radical in each case can also carry a methyl radical,
1-hydroxy-4-methyl-6-(2-bicyclo[2,2,1]heptyl)-2-pyridone,
1-hydroxy-3,4-dimethyl-6-benzyl- or -6-dimethylbenzyl-2-pyridone or
1-hydroxy-4-methyl-6-(β-phenylethyl)-2-pyridone.

The above-mentioned compounds of the formula I can be employed both in free form and as salts; use in free form is preferred.

If organic bases are used, poorly volatile bases are preferably employed, for example low molecular weight alkanolamines such as ethanolamine, diethanolamine, N-ethylethanolamine, N-methyldiethanolamine, triethanolamine, diethylaminoethanol, 2-amino-2-methyl-n-propanol, dimethylaminopropanol, 2-amino-2-methylpropanediol, triisopropanolamine. Further poorly volatile bases which may be mentioned are, for example, ethylenediamaine, hexamethylenediamine, morpholine, piperidine, piperazine, cyclohexylamine, tributylamine, dodecylamine, N,N-dimethyldodecylamine, stearylamine, oleylamine, benzylamine, dibenzylamine, N-ethylbenzylamine, dimethylstearylamine, N-methylmorpholine, N-methylpiperazine, 4-methylcyclohexylamine, N-hydroxyethylmorpholine. The salts of quaternary ammonium hydroxides such as trimethylbenzylammonium hydroxide, tetramethylammonium hydroxide or tetraethylammonium hydroxide can also be used, and furthermore guanidine and its derivatives, in particular its alkylation products. However, it is also possible to employ, for example, low molecular alkylamines such as methylamine, ethylamine or triethylamine as salt-forming agents. Salts with inorganic cations, for example alkali metal salts, in particular sodium, potassium or ammonium salts, alkaline earth metal salts such as in particular the magnesium or calcium salts, and salts with di- to tetravalent cations, for example the zinc, aluminium or zirconium salt, are also suitable for the compounds to be employed according to the invention.

The active compounds of the formula I to be employed in the preparations can be prepared, for example, by the process according to US 2 540 218 or US 4 797 409.

The present invention also relates to the use of the compound of the formula I, in which Ar is a bicyclic system, which is derived from biphenyl, diphenylalkane or diphenyl ether.

The present invention also relates to the use of the compound of the formula I, which contains a cyclohexyl radical in the position R⁴.

The present invention also relates to the use of the compound of the formula I, which contains an octyl radical of the formula -CH₂-CH(CH₃)-CH₂-C(CH₃)₃ in the position R⁴.

The present invention also relates to the use of the compound of the formula I, wherein 1-hydroxy-4-methyl-6-[4-(4-chlorophenoxy)phenoxymethyl]-2-(1H)pyridone, 1-hydroxy-4-methyl-6-cyclohexyl-2-(1H)pyridone or 1-hydroxy-4-methyl-6-(2,4,4-trimethylpentyl)-2-(1H)pyridone is employed.

The present invention also relates to the use of the compounds of the formula I for the production of a pharmaceutical for the treatment of wounds.

Due to the induction of angiogenesis the healing of wounds is improved. Wounds are e.g. cuts, gashs, chaps, sores, gaping wounds, stab wounds, gunshot wounds, squeeze wounds, crush wounds, scratches, skin burns, scorch wounds, diabetic foot ulcers, pressure ulcers, venous ulcers, leg ulcers, wounds due to surgery, plastic surgery, transplantation or grafting of organs.

The compounds of the formula I and their physiologically tolerable salts can be administered to animals, preferably to mammals, and in particular to humans as pharmaceuticals for the treatment of wounds and/or induction of angiogenesis. They can be administered on their own, or in mixtures with one another or in the form of pharmaceutical preparations which permit topical administration.

The pharmaceuticals can be administered percutaneously or topically, for example in the form of emulsions, ointments, solutions or tinctures, powders, or in other ways, for example in the form of aerosols or foams. For use according to the invention of the compounds mentioned, liquid, semisolid and solid pharmaceutical preparations are suitable, in particular solutions, cream, ointment, powders and gel preparations, where the latter are preferably used because of their increased release of active compound.

The pharmaceutical preparations according to the invention are prepared in a manner known per se and familiar to one skilled in the art, pharmaceutically acceptable inert inorganic and/or organic carriers being used in addition to the compound(s) of the formula I and/or its (their) physiologically tolerable salts.

The compounds of the formula I can also be incorporated in or on surgical gauze, bandage or dressing.

Suitable dressings are illustrated in more detail by the following examples:

### Alginate dressings

Alginate wound dressings are woven from calcium alginate fibers. Calcium alginate is a marine biopolymer that is derived from seaweed. Alginates are highly absorptive. When the alginates dressing comes into contact with wound exudate, a soft gel is formed, maintaining a moist environment at the wound surface that is conducive to the formation of granulation tissue and epithelialization. Alginate dressings are indicated for heavily exudating wounds, as they have the capacity to absorb as much as 20 times their weight.

### Composite dressings

They are generally multi-layered, incorporating a nonadherent layer that is in contact with the wound, and various absorbent, wicking, and semi-occlusive layers. The absorptive layer does not incorporate a foam, alginate, hydrocolloid, or hydrogel. A nonwoven adhesive tape backing secures the dressing to the skin. These dressings are used as cover dressings for all phases of wound healing and are primary dressing for many types of post-operative wounds.

### Contact layers

Contact layers are thin sheet dressings placed directly on an open wound to protect the wound tissue from direct contact with other agents or dressings applied to the wound. Secondary dressings are always used with contact layers.

### Foam dressings

Foam dressings are made primarily from polyurethane, but silicone is also used. They are available as either flat foam dressings or as fillers, and are intended for use on granulation and epithelializing wounds producing some exudate.

### Gauze

Woven gauze dressings are made from 100% cotton yarn. The threads are horizontally and vertically woven, with 10 to 20 separate manufacturing steps employed in the process of producing a 4 x 4 inch, 12-ply sponge. Woven gauze dressings come in a wide range of types and are commonly used for packing and debridement.
Nonwoven fabrics are neither woven nor knitted. The fibers are arranged such that they appear to be woven. Most nonwoven dressings are manufactured from a blend of rayon and polyester. Rayon provides softness and absorbency, while polyester provides strength. The primary application of nonwoven gauze dressings is for absorption and wrapping.

### Impregnated gauze dressings

Impregnated gauze dressings are woven or nonwoven materials in which substances have been incorporated into the dressing material.

### Hydrocolloid dressings

Hydrocolloid dressings are popular second-generation dressings, which are moderate absorptive, moisture-retentive, conformable to the wound, and self-adhesive. They are also occlusive, forming a barrier against bacterial contamination. In addition to being available in dimensional forms, hydrocolloids are supplied as powders, granules, pastes, and other specialised forms. The major functional components are gelatine, pectin, or related hydrocolloids. They are indicated for use on pressure ulcers, burns, and a variety of other wound types.

### Hydrogel dressings

Hydrogels are semipermeable dressings of which water is the major component. Glycerine or propylene glycol are included as humectants, and a co-polymer starch is sometimes added as absorptive. The dressings are available as gauze-impregnated wafers, sheets, and amorphous gels, and are indicated for partial- and full-thickness wounds non-draining to moderately draining.

### Transparent film dressings

Transparent film dressings are occlusive dressings consisting of a polyurethane membrane framed by an adhesive layer. The dressings vary in thickness, degree of occlusiveness, and other properties. Transparent film dressings are indicated for use on small surgical incisions, insertion points for peripheral and central venous catheters, superficial burns, partial-thickness breakdown wounds, and as secondary dressings.

### Biological and biosynthetic dressings

This sector includes
- a group of naturally absorptive and biological compatible collagen-based dressings
- dressings constructed of related biocompatible amino acids and protein hydrolysates
- dressings which combine synthetic and biologically derived materials

The pharmaceutical preparations normally contain from about 0.05 % to about 5 %, preferably from 0.5 % to 2 %, especially preferably 1%, by weight of the compounds of the formula I and/or their physiologically tolerable salts. Smaller and higher percentages are also possible.

In addition to the active ingredients of the formula I and/or their physiologically acceptable salts and to carrier substances, the pharmaceutical preparations can contain additives such as, for example, fillers, binders, lubricants, wetting agents, stabilizers, emulsifiers, preservatives, colorants, thickeners, diluents, buffer substances, solvents, solubilizers, agents for achieving a depot effect, salts for altering the osmotic pressure, coating agents or antioxidants. They can also contain two or more compounds of the formula I and/or their physiologically tolerable salts. Furthermore, in addition to at least one compound of the formula I and/or its physiologically tolerable salts, the pharmaceutical preparations can also contain one or more other therapeutically or prophylactically active ingredients. Such additional active ingredients can be dexpanthenol, allantoin, dextranomer, and/or extracts of the Echinacea plant.

It is understood that changes that do not substantially affect the activity of the various embodiments of this invention are included within the invention disclosed herein. Thus, the following examples are intended to illustrate but not limit the present invention.

### Example 1

### Induction of VEGF gene expression by CPX

### Methods

### Cell culture

The HRCHO5 cells containing a stable transfected, HIF-1-dependent reporter gene were described previously (Wanner, R. M., et al. (2000) Epolones induce erythropoietin expression via hypoxia-inducible factor-1α activation. *Blood* **96,** 1558-1565 ). The human HepG2 hepatoma cell line was obtained from American Type Culture Collection (ATCC numbers HB-8065). All cell lines were cultured in Dulbecco's modified Eagle's medium (DMEM, high glucose, Life Technologies) supplemented with 10% heat-inactivated FCS, 100 U/ml penicillin, 100 µg/ml streptomycin, 1 x non-essential amino acids and 1 mM Na-pyruvate (all purchased from Life Technologies) in a humidified atmosphere containing 5% CO₂ at 37°C. Oxygen partial pressures in the incubator (Forma Scientific, model 3131) were either 140 mm Hg (20% O₂ v/v, normoxia) or 7 mm Hg (1% O₂ v/v, hypoxia). Ciclopirox olamine (CPX), deferoxamine mesylate (DFX) and 2,2'-dipyridyl (DP) were purchased from Sigma. Stock solutions of CPX and DP were prepared in methanol at 0.5 M and 1 M, respectively. DFX was freshly dissolved at 10 mM into the cell culture medium.

### Transient transfection, reporter gene and viability assays

HepG2 cells were transiently electroporated with a VEGF promoter-driven luciferase construct as described previously (Ikeda, E., et al. (1995) Hypoxia-induced transcriptional activation and increased mRNA stability of vascular endothelial growth factor in C6 glioma cells. *J. Biol. Chem.* **270,** 19761-1976628). Following stimulation, transiently and stable transfected cells were lysed in passive lysis buffer (Promega) and the luciferase activity was determined using a luciferase assay kit according to the manufacturer's instructions (Promega) in a 96-well luminometer (MicroLumat LB96P, EG&G Berthold). Cell proliferation/viability was assessed by the 3-(4,5-dimethylthiazol-2-yl)-2,5-diphenyl tetrazolium bromide (MTT) assay as described (30, 31). Absorbances at 570 nm were determined in a 96-well photometer (Rainbow, SLT Labsystems).

### Results

CPX in a concentration from 5 µM to 20 µM concentrations strongly induced the HIF-1α protein in normoxic HepG2 hepatoma cells after 6 and 24 hours of treatment. While no HIF-1α protein could be detected in normoxic cells, hypoxia (1% O₂) also strongly induced HIF-1α. The combination of CPX and hypoxia did not further increase HIF-1α levels after 6 hours but rather decreased HIF-1α levels after 24 hours of treatment.

As determined by Northern blotting of mRNA derived from HepG2 cells treated with CPX, DFX and DP for 24, 48 and 72 hours, these iron chelators also induced the expression of the endogenous HIF-1 target genes VEGF, glucose transporter-1 (Glut-1) and aldolase but not of the control genes L28 and β-actin.

The functionality of CPX-induced VEGF mRNA expression was further demonstrated by the accumulation of VEGF protein in the supernatant of the HepG2 cell culture, which increased in a dose-dependent manner following 6 or 24 hours of treatment.

A luciferase reporter gene driven by a 1180 bp VEGF promoter fragment was transiently transfected into HepG2 cells, which were split and treated with increasing concentrations of CPX for 24 hours. A dose-dependent increase in luciferase activity was observed, suggesting that CPX transcriptionally activates VEGF expression. In summary, these results demonstrate that CPX can activate endogenous VEGF transcription, mRNA expression and secreted protein accumulation.

### Example 2

### Lack of HIF-1 target gene induction by CPX in the isolated perfused rat kidney

Systemically administered DFX can induce ubiquitous HIF-1 stability and kidney-specific erythropoietin expression in mice (Wang, G. L., and Semenza, G. L. (1993) Desferrioxamine induces erythropoietin gene expression and hypoxia-inducible factor 1 DNA-binding activity: implications for models of hypoxia signal transduction. *Blood* **82,** 3610-3615 ). Regarding a topical application of CPX as a HIF-1-inducing agent for therapeutic angiogenesis, it was important to test whether CPX could lead to a systemic induction of VEGF. As an *ex vivo* model, isolated rat kidneys were perfused for 3 hours under normoxic conditions with increasing concentrations of CPX. However, CPX did not induce the mRNA levels of the HIF-1 target genes VEGF, Glut-1 or aldolase at concentrations ranging from 0.1 µM to 100 µM. The higher CPX concentrations (33 µM and 100 µM) led to a down regulation of kidney function as indicated by a strong increase in the albumin and sodium excretion rates. Following perfusion with 3% oxygen for 3 hours, an increase in Glut-1 but not in VEGF and aldolase mRNA levels was observed which was not further elevated by CPX.

### Example 3

### Induction of angiogenesis in the chicken chorioallantoic membrane (CAM) by CPX Chick embryo chorioallantoic membrane (CAM) assay

### Method

CAM assays were performed as described by Olivo, M. et al. (A comparative study on the effects of tumor necrosis factor-α (TNF-α), human angiogenic factor (h-AF) and basic fibroblast growth factor (bFGF) on the chorioallantoic membrane of the chick embryo, (1992). *Anat. Rec.* **234,** 105-115). Briefly, fertilized eggs (purchased from Lohmann Tierzucht, Cuxhaven, Germany) were incubated at 37 °C in a humidified atmosphere. The eggs were kept on their sides and turned upside down twice a day. After 6 days, a small hole was drilled through the shell into the air sac (visualized using a strong light source) and a window of approximately 0.5 cm² was sawed into the side of the egg. The window was sealed with tape and the eggs were reincubated until day 9, when 6-Cyclohexyl-1-hydroxy-4-methyl-2(1H)-pyridone 2-aminoethanol salt (CPX) was applied to the CAM using the Elvax® method (see Olivo, M. et al. above). Therefore, ethylene/vinyl acetate copolymer beads (Elvax® 40L-03, DuPont, provided by C. H. Erbslöh KG, Krefeld, Germany) were extensively washed in ethanol and dried under reduced pressure. The Elvax® beads were then dissolved in methylene chloride at 10% (w/v) and CPX was added to the desired concentration. One drop (40 µL) of this solution was pipetted onto a siliconized glass slide and the solvent was allowed to evaporate completely. Using forceps, the Elvax® disc was carefully lifted from the glass slides and placed onto the CAM. At day 11, the window was enlarged and the CAM was documented using a digital camera (Olympus) coupled to an ocular of a stereomicroscope.

### Result

Based on the observations that CPX induced VEGF expression in cell culture (see example 1), the angiogenic capacity in an *in vivo* model of angiogenesis was analyzed. Therefore, the chicken CAM at day 9 of development was overlayed with inert polymer discs containing various concentrations of CPX or solvent only. After two days of incubation, no signs of angiogenesis could be observed with the solvent control discs. In contrast, polymer discs containing 50 mM CPX consistently induced CAM angiogenesis as evidenced by numerous newly formed, radially arranged vessels. A total of 10 CAMs treated with 50 mM CPX showed similar angiogenesis, while all 7 control CAMs lacked any signs of angiogenesis. 5 mM CPX was indistinguishable from the controls and 500 mM CPX was toxic to the chick embryo.

### Example 4

### Mouse skin wound model

### Method

Both ears of adult NMRI mice were wounded under anesthesia with 100 mg/kg Ketamine (Narketan®) and 5 mg/kg Xylazine (Rompun®) by punch-through holes of 2 mm diameter using a standard ear-punching tool. The ears were treated daily with a common skin cream containing (Batrafen®) or not containing 1 % (w/v) CPX. One ear was treated with CPX and the other ear with the control cream. The amount of cream was sufficient to cover the hole and was equally distributed over the entire ear.

### Result

### CPX can induce angiogenesis in a mouse skin wound model

Punch-through ear holes of 2 mm diameter (a widespread way of marking mice in animal facilities) were treated daily with a commercially available dermal cream containing 1% CPX on one ear and a CPX-free but otherwise identical common dermal cream on the other ear, respectively. CPX can cause reddening of the wound margin, which was never observed on the healthy skin area of the same ear. In this series 4 of 10 animals showed similar effects and none of the animals had more pronounced reddening on the placebo-treated wound margin than on the CPX-treated wound margin. The reddening induced by CPX might be enhanced vessel growth.

## Claims

1. The use of 1-hydroxy-2-pyridones of the formula I 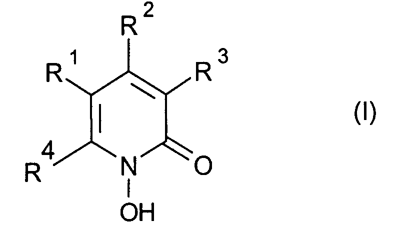 for the preparation of a pharmaceutical for the induction of angiogenesis,
in which R¹, R² and R³ are identical or different and are a hydrogen atom or alkyl having 1-4 carbon atoms, and
R⁴ is a saturated hydrocarbon radical having 6 to 9 carbon atoms or a radical of the formula II 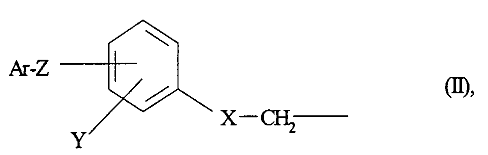 where
X is S or O,
Y is a hydrogen atom or up to 2 halogen atoms such as chlorine and/or bromine,
Z is a single bond or the divalent radicals O, S, -C(R⁵)(R⁶)-; wherein R⁵ and R⁶ are identical or different and are hydrogen atom or (C₁-C₄)-alkyl; or other divalent radicals having 2-10 carbon and optionally O and/or S atoms linked in the form of a chain, where - if the radicals contain 2 or more O and/or S atoms - the latter must be separated from one another by at least 2 carbon atoms and where 2 adjacent carbon atoms can also be linked to one another by a double bond and the free valences of the carbon atoms are saturated by hydrogen atom and/or (C₁-C₄)-alkyl groups,
Ar is an aromatic ring system having up to two rings which can be substituted by up to three radicals from the group consisting of fluorine, chlorine, bromine, methoxy, (C₁-C₄)-alkyl, trifluoromethyl and trifluoromethoxy in free or in salt form.

2. The use as claimed in claim 1, wherein
Ar in the compound of the formula I is a bicyclic system, which is derived from biphenyl, diphenylalkane or diphenyl ether.

3. The use as claimed in claim 1 or 2, wherein the compound of formula I contains a cyclohexyl radical in the position R⁴.

4. The use as claimed in one or more of claims 1 to 3, wherein the compound of formula I contains an octyl radical of the formula -CH₂-CH(CH₃)-CH₂-C(CH₃)₃ in the position R⁴.

5. The use as claimed in one or more of claims 1 to 4, wherein the compound of formula I is 1-hydroxy-4-methyl-6-[4-(4-chlorophenoxy)phenoxymethyl]-2-(1H)pyridone, 1-hydroxy-4-methyl-6-cyclohexyl-2-(1H)pyridone or 1-hydroxy-4-methyl-6-(2,4,4-trimethylpentyl)-2-(1H)pyridone.

6. The use of a compound of the formula I as claimed in one or more of claims 1 to 5, for producing a pharmaceutical for the treatment of wounds.

7. The use as claimed in claim 6, wherein the wounds are cuts, gashs, chaps, sores, gaping wounds, stab wounds, gunshot wounds, squeeze wounds, crush wounds, scratches, skin burns, scorch wounds, diabetic foot ulcers, pressure ulcers, venous ulcers, leg ulcers, wounds due to surgery, plastic surgery or transplantation or grafting of organs.

8. The use as claimed in one or more of claims 1 to 7, wherein the compound of formula I is administered as their own, or in mixtures with one another or in the form of pharmaceutical preparations which permit topical administration.

9. The use as claimed in claim 8, wherein the compound of the formula I is administered percutaneously or topically in the form of emulsions, ointments, solutions or tinctures, powders or in the form of aerosols or foams.

10. The use as claimed in claims 8 or 9, wherein the pharmaceutical preparation is liquid, semisolid and solid and is a solution, cream, ointment, powder or gel preparation.

11. The use as claimed in one or more of claims 1 to 10, wherein the compound of formula I is incorporated in or on surgical gauze, bandage or dressing.

12. The use as claimed in claim 11, wherein the compound of the formula I is incorporated on or in alginate dressings, composite dressings, contact layers, foam dressings, gauzes, impregnated gauze dressings, hydrocolloid dressings, hydrogel dressings, transparent film dressings or biological and biosynthetic dressings.

13. The use as claimed in one or more of claims 1 to 12, wherein the dose of the compound of formula I in the pharmaceutical preparation is from about 0.05 % to about 5 %, preferably from 0.5 % to 2 %, especially preferably 1 % , by weight of the compound of the formula I and/or their physiologically tolerable salts.

14. The use as claimed in one or more of claims 1 to 13, wherein the pharmaceutical preparation contains in addition an active ingredient such as dexpanthenol, allantoin, dextranomer or extracts of the Echinacea plant or a mixture of said ingredients.
